(19) Europäisches Patentamt
European Patent Office
Office européen des brevets

(11) Veröffentlichungsnummer: **0 454 185 A2**

(12) # EUROPÄISCHE PATENTANMELDUNG

(21) Anmeldenummer: **91111514.5**

(51) Int. Cl.5: **A61K 49/00**

(22) Anmeldetag: **14.10.88**

This application was filed on 10.07.1991 as a divisional application to the application mentioned under INID code 60.

(30) Priorität: **15.10.87 SU 4313068**

(43) Veröffentlichungstag der Anmeldung: **30.10.91 Patentblatt 91/44**

(60) Veröffentlichungsnummer der früheren Anmeldung nach Art. 76 EPÜ: **0 313 942**

(84) Benannte Vertragsstaaten: **CH DE FR GB IT LI SE**

(71) Anmelder: **Bio-Photonics, Inc.**
**10641 First Street East**
**Treasure Island Florida 33706(US)**

(72) Erfinder: **Dzbanovsky, Nikolai Nakolaevich**
**ulitsa V. Ulbrikhta, 8, kv.95**
**Moscow(SU)**
Erfinder: **Polsachev, Viktor Iosifovich**
**Izmailovsky prospekt, 123/I, kv.29**
**Moscow(SU)**
Erfinder: **Potemkina, Elena Vasilievna**
**Frunzenskaya naberezhnaya 8, kv.26**
**Moscow(SU)**
Erfinder: **Rakhimov, Alexandr Tursonovich**
**Rostovskaya naberezhnaya I, kv.95**
**Moscow(SU)**
Erfinder: **Rubin, Leonid Borisovich**
**Lomonosovsky prospekt, 14, kv.499**
**Moscow(SU)**
Erfinder: **Osipov, Alexandr Sergeevich**
**Mosfilmovskaya ulitsa, 32, kv.42**
**Moscow(SU)**

(74) Vertreter: **von Füner, Alexander, Dr. et al**
**Patentanwälte v. Füner, Ebbinghaus, Finck**
**Mariahilfplatz 2 & 3**
**W-8000 München 90(DE)**

(54) **Verwendung von Fluoronderivaten zur Kontrastierung von bösartigen Neubildungen bei deren Diagnostik.**

(57) Die Erfindung bezieht sich auf die Verwendung von Fluoronderivaten der allgemeinen Formel I in der Onkologie für die Diagnostik, darunter auch für die Frühdiagnostik von bösartigen Neubildungen, für die Bestimmung der Verbreitung einer bösartigen Schädigung, Bestimmung der Diagnose in Kliniken und Gesundheitsfürsorgestellen, die Überwachung der Behandlung von bösartigen Geschwulsten und die Feststellung von Nachoperationsrückfällen bei einer Fluoreszenz- und der NMR-Kontrastierung von bösartigen Neubildungen.

EP 0 454 185 A2

Die vorliegende Erfindung bezieht sich auf die Medizin, und zwar auf die Onkologie.

Am effektivsten kann die vorliegende Erfindung für die Diagnostik, darunter auch für eine Frühdiagnostik von bösartigen Neubildungen, für die Bestimmung der Verbreitung der bösartigen Schädigung, die Bestimmung des Umfangs der Operationen, die Präzisierung der Diagnose in Kliniken und Gesundheitsfürsorgestellen, die Überwachung der Behandlung von bösartigen Geschwulsten und die Feststellung von Nachoperationsrückfällen angewendet werden.

Gegenwärtig stellt das Problem, bösartige Neubildungen, insbesondere in frühen Stadien, zu diagnostizieren, eines der aktuellsten Probleme in der Onkologie dar. Trotz einer breiten Anwendung von histologischen Methoden der Analyse, den großen Erfolgen der Ultraschall- und Röntgendiagnostik, der Endoskopieuntersuchungen sowie der Röntgen- und der NMR-Tomografie sind die Empfindlichkeit, die Genauigkeit und die Zugänglichkeit dieser Methoden nicht ausreichend. Eine der Richtungen für die Vervollkommnung der Diagnostik von bösartigen Geschwulsten besteht in der Anwendung von darin selektiv akkumulierten konstrastierenden Stoffen. Es ist z.B. gut bekannt, daß die bösartigen Geschwulste einige Farbstoffe in einer erhöhten Konzentration gegenüber den normalen Geweben akkumulieren, was im Prinzip für eine Fluoreszenzdiagnostik der Geschwulst nach einer charakterstischen Fluoreszenz des darin akkumulierten Farbstoffes angewendet werden könnte. Das für diese Zwecke verwendete Fluoreszein gab keine positiven Ergebnisse, da der Kontrast der Akkumulierung des Fluoreszeins im bösartigen Gewebe gegenüber dem normalen Gewebe für eine zuverlässige Diagnostik einer bösartigen Schädigung nicht ausreichend war (s. Ju.N. Efuni, Westnik otorinolaringologii, 1961, Nr. 2, S. 11-15).

Der Akkumulierungskontrast eines beliebigen fluoreszierenden kontrastierenden Stoffes in einer bösartigen Geschwulst wird im Vergleich zu einem normalen Gewebe durch das Verhältnis der Konzentrationen des darin enthaltenen Stoffes bestimmt.

Es ist ein Mittel zur Kontrastierung von bösartigen Geschwulsten bei der Diagnostik bekannt, welches aus einer wässerigen Lösung von Hämatoporphyrinderivaten besteht (s. Hematoporphyrin Derivative photoradiation Therapy of Cancer, M., Berns Ed. Alan R. Liss Inc. NY 1984; G.H.M. Gijsbers, D. Breederveld, M.I.C. van Gemert, T.A. Boon, S. Langelaar, R.P.H. Rettschnick. Laser in the life sciences I, Nr. 1, 29-49, 1986).

Die Nachteile des Hämatoporphyrins als eines kontrastierenden Stoffes sind folgende: eine niedrige Selektivität der Akkumulierung in bösartigen Geweben gegenüber den normalen Geweben (der Akkumulierungskontrast beträgt etwa 4 relative Einheiten); die Notwendigkeit, diesen Stoff in hohen Konzentrationen anzuwenden; die Toxizität und das Vorhandensein negativer Nebeneffekte (z.B. eine fotodynamische Schädigung der Haut) auf den Organismus der Labortiere, der Zellkulturen und der Menschen.

Als einen weiteren Nachteil dieses Stoffes kann man bezeichnen, daß es damit unmöglich ist, Geschwulste der inneren Organe und Metastasen, z.B. in der Leber, in Knochen und im Gehirn zu diagnostizieren, weil Hämatoporphyrin nur für eine Fluoreszenzkontrastierung der für eine optische Untersuchung zugänglichen Geschwulste, d.h. der Geschwulste, die entweder auf dem Körper selbst oder an der Innenfläche der Organe (Lungen, Verdauungssystem, Bauchhöhle u.a.) lokalisiert sind, angewendet werden kann.

Außerdem besteht der Nachteil dieses Stoffes darin, daß das Hämatopyrphyrin in seiner Zusammensetzung keine chemischen Elemente enthält, die radioaktive Isotope haben, was es unmöglich macht, diesen Stoff für eine Radioisotopendiagnostik anzuwenden.

Aus JP-A-60 171 436 ist die Anwendung von Eosin gelb durch interarterielle Injektion in ein Krebsgewebe zur Kontrastierung bekannt.

Aus O.A. Neumüller: "Römpps Chemie-Lexikon", 1981, Seite 1336, Frankh'sche Verlagshandlung, Stutgart, DE, ist zu entnehmen, daß Fluroescein fluoresziert und als Indikator zur Diagnose, beispielsweise von Hornhautschäden, eingesetzt wird.

Die FR-A-3 820 M betrifft ein Medikament auf der Basis von Hydroxyfluoran als Kontrastierungsmittel im Magen.

Der vorliegenden Erfindung liegt die Aufgabe zugrunde, Stoffe zu finden, welche die oben genannten Nachteile bei der Kontrastierung bösartiger Neubildungen unabhängig von ihrer Lokalisation beseitigen.

Die gestellte Aufgabe wurde dadurch gelöst, daß ein Kreis von Derivaten der allgemeinen Formel I bestimmt wurde,

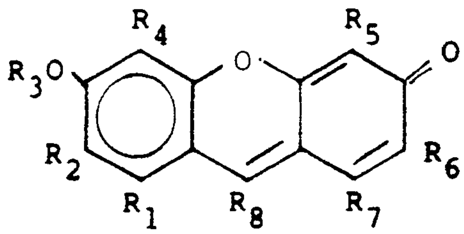

(I)

worin

$R_1$ und $R_7$ F oder einer F und der andere Wasserstoff (H), Cl, Br, J , $CH_3$, $CF_3$, $CCl_3$, $C_2H_5$, COOH, $COOCH_3$, $NO_2$,$NH_2$, OH, $OCH_3$ oder $OC_2H_5$ bedeuten;

$R_2$, $R_4$, $R_5$ und $R_6$ alle F oder eins bis drei F und der Rest eines oder mehrere aus der Gruppe Wasserstoff (H), Cl, Br, J, A, B, W, G, AW, AG, BW, BG, $CH_3$, $CF_3$, $CCl_3$, $CBR_3$, $CJ_3$, COOH, COOA, COOB, OA, OB, $NO_2$ und $NH_2$ bedeuten,

$R_3$ Wasserstoff (H), das Kation eines Alkalimetalls oder Ammonium, A, B, W, G, Q, Z oder Glukoronsäure bedeutet;

$R_8$ Wasserstoff (H) oder F oder F, welches in beliebiger Kombination in den Gruppen A, B, W, G, AW, AG, BW, BG, I, K, AI, BI, BK, AK, WI, WK, GI, GK, L, M, LM, Y, V, AY, BY, BV, AV, WY, GY, WV und GV vorliegt, werden für eine NMR-Kontrastierung von bösartigen Neubildungen ebenfalls bevorzugt.

Bevorzugter ist die Verwendung von Fluoronderivaten der allgemeinen Formel I, worin

$R_1$ und $R_7$ F oder einer F und der andere Wasserstoff (H), Cl, $CH_3$, $CF_3$, $NO_2$ oder $NH_2$ bedeuten;

$R_2$, $R_4$, $R_5$ und $R_6$ alle F oder eins bis 3 F und der Rest eins oder mehrere aus der Gruppe Wasserstoff (H), $CF_3$, $NO_2$, $NH_2$ und OH bedeuten;

$R_3$ Wasserstoff (H), Kation eines Alkalimetalls oder Ammonium bedeutet;

$R_8$ F oder die Gruppe A, substituiert durch F von eins bis zur vollständigen Substitution, die Gruppe B, substituiert durch F von eins bis zur vollständigen Substitution, die Gruppe I, substituiert durch F von eins bis zur vollständigen Substitution, die Gruppe Y, substituiert durch F, $CF_3$ von eins bis zur vollständigen Substitution, bei beliebiger Kombination unter ihnen, für eine NMR-Kontrastierung von bösartigen Neubildungen.

Fluoronderivate der allgemeinen Formel I, worin

$R_1$ und $R_7$ jeder mindestens eines aus der Gruppe Wasserstoff (H), F, Cl, $CH_3$,$CF_3$, $CCl_3$, COOH, $COOCH_3$, $NO_2$, $NH_2$, OH, $OCH_3$ oder $OC_2H_5$ bedeuten;

$R_2$, $R_4$, $R_5$ und $R_6$ aus der Gruppe Wasserstoff (H), F, Cl, die Gruppe A, die Gruppe A, substituiert durch F, Cl, von eins bis zur vollständigen Substitution bei beliebiger Kombination unter ihnen, $CH_3$, $CF_3$, $CCl_3$, COOH, COOA, COOB, OA, OB, $NO_2$, $NH_2$ und W bedeuten;

Gruppe X - die Gruppe W, substituiert von eins bis zur vollständigen Substitution durch F, Cl, $NO_2$, $NH_2$, OH, $HSO_3^-$ , COOH oder $CO^-$ bedeutet;

$R_3$ Wasserstoff (H), Kation eines Alkalimetalls oder Ammonium, A, P, W, X, Q oder Acyl (R-CH=O) bedeutet;

$R_8$ Wasserstoff (H), A, P, W, X, AW, AX, PW, PX und I, Gruppe T die Gruppe I, substituiert durch F, Cl, $NO_2$, $NH_2$, OH, $HSO_3^-$ , COOH, $COO^-$, von eins bis zur vollständigen Substituierung bei beliebiger Kombination unter ihnen, AI, PI, AT, PT, WI, XI, WT, XT, L, M, LM, Y, Gruppe U die Gruppe Y, substituiert durch $NO_2$, $NH_2$, OH, COOH, $COO^-$, $HSO_3^-$ , F, Cl, COOA, COOX,

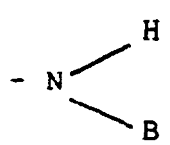 , 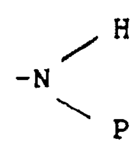 ,

$CH_3$, $CF_3$, $CCl_3$ von eins bis zur vollständigen Substitution in beliebiger Kombination unter ihnen, AY, PY, AU, PU, WY, XU, XY oder WU ist, sind vorzüglich für eine Fluoreszenzkontrastierung von bösartigen Neubildungen geeignet.

Die Anwendung von Fluoronderivaten der allgemeinen Formel I, worin $R_1$ und $R_7$ aus der Gruppe Wasserstoff (H), F, Cl, $NO_2$, $NH_2$ und OH ausgewählt sind;

$R_2$, $R_4$, $R_5$ und $R_6$ aus der Gruppe Wasserstoff (H), F, Cl, $NO_2$, $NH_2$ und OH bedeuten;

$R_3$ Wasserstoff (H), das Kation eines Alkalimetalls oder Ammonium bedeutet;
$R_8$ Wasserstoff (H), die Gruppe W, X, I, T, Y oder U ist, ist für eine Fluoreszenzkontrastierung von bösartigen Neubildungen besonders bevorzugt.

Die Verbindungen der oben angeführten Formel weisen eine Fähigkeit auf, sich selektiv in großen Konzentrationen in lebendigen bösartigen Geschwulsten statt in normalen Zellen und Geweben zu akkumulieren.

Es ist zweckmäßig, die Fluoronderivate in einer Menge von 0,1 bis 20 mg je 1 kg der Masse eines lebendigen Organismus anzuwenden. Die untere Grenze der Konzentrationen wird durch die Empfindlichkeit der Methode zur Registrierung der Fluoronderivate in Geweben und Zellen bestimmt. Die obere Grenze der Konzentrationen ist mit der begrenzten Lösbarkeit der Fluoronderivate verbunden. Die Ergebnisse der Versuche an Labortieren haben gezeigt, daß im Bereich der Konzentrationen von 0,1 bis 20,0 mg/kg der Masse die Fluoronderivate keine toxische Wirkung ($LD_{50}$ > 300 mg/kg der Masse) hervorrufen.

Man kann das Mittel zur Kontrastierung von bösartigen Neubildungen durch intravenöse Injektionen, enteral oder über den Anus einführen.

Als Lösungsmittel für das Fluoronderivat kann Wasser oder eine 5 bis 30%ige wässerige ethanolische Lösung angewendet werden.

In Abhängigkeit von der Lösbarkeit der Fluoronderivate können auch Öllösungen-Solutio oleosa - und alkoholische Zuckerlösungen - Solutio spirituosa-saccharum - angewendet werden. Die öligen Lösungen werden auf der Grundlage von Rizinus-, Oliven-, Sonnenblumen-, Baumwollsamen-, Pfirsichöl und der anderen Öle, die für eine enterale Einführung in der Pharmakologie angewendet werden, hergestellt.

Das Fluoronderivat gewährleistet eine hohe Kontrastierung von bösartigen Geschwulsten durch seine selektive Akkumulierung in erhöhten Konzentrationen in diesen Geschwulsten gegenüber den normalen Geweben. Der Einsatz der Fluoronderivate mit einem hohen Austritt der Fluoreszenz ist optimal für eine Fluoreszenzkontrastierung von bösartigen Geschwulsten. Die Anwendung der Fluorderivate von Fluoron gewährleistet eine Kontrastierung bösartiger Geschwulste bei NMR-Tomografieuntersuchungen; dabei werden Geschwulste beliebiger Lokalisation entdeckt.

Die Fluoronderivate werden in an sich bekannter Weise erhalten, z.B. durch eine ein- oder zweistufige Kondensation von Phthalsäureanhydrid mit Resorzinderivaten oder durch Kondensation von verschiedenen Resorzinderivaten und Aldehyden sowie durch eine Behandlung der Xanthonfarbstoffe mit entsprechenden Reaktanten.

Die Bestimmung der Fähigkeit der Fluoronderivate, sich in bösartigen Zellen und Geschwulsten zu akkumulieren, wurde auf Zellkulturen der He-, Za-, ZTZ-, Z-Fibroplaste, welche nach Standardmethoden kultiviert werden, durchgeführt. Nachdem der Zellkultur ein Fluoronderivat neben den anderen Komponenten des Mittels zugesetzt und innerhalb eines bestimmten Zeitabschnitts inkubiert worden war, wurde eine Zellenfraktion entnommen und nach dem Waschen in den Zellen die Konzentration des Fluoronderivats unter Anwendung der Methoden einer chromatografischen und einer Fluoreszenzanalyse bestimmt.

In Versuchen mit Labortieren wurden Mäuse CBA, C-5EB, Black und BALB-C mit umgeimpften Geschwulsten carciona cervix, Lewis tumor, carciona intestinum crassum getestet. Nach einer bestimmten Zeit nach der Einführung der Fluoronderivate neben den anderen Komponenten des Mittels wurden die Tiere unter Narkose getötet und die Konzentration des Fluoronderivats in der Geschwulst und in normalen Geweben bestimmt. Der Kontrast der Akkumulierung der Fluoronderivate wurde als Verhältnis der Konzentrationen des Präparats im bösartig/normalen Gewebe bestimmt.

Bei der Anwendung von Fluorderivaten des Fluorons wurden die Methoden der Fluoreszenz- und NMR-Tomografie für die Bewertung der kontrastierenden Fähigkeit der Präparate angewendet.

Bei laparoskopischen Untersuchungen wurde festgestellt, daß sich die Fluoronderivate in der Aszitflüssigkeit akkumulieren, was es gestattet, diese in sehr geringen Mengen zu entdecken und zu dem Schluß zu kommen, daß es in Organen bösartige Tiefmetastasen, die nicht visuell entdeckt werden können, z.B. in der Leber, im Bauchgebiet, in der Milz u.a. gibt, was diagnostische Möglichkeiten der Laparoskopie wesentlich erweitert.

Die im Blut anwesenden bösartigen Elemente akkumulieren die Fluoronderivate und können nach der Kontrastierung, z.B. einer Fluoreszenzkontrastierung unter dem Mikroskop entdeckt werden.

Man beginnt die Untersuchung der Lokalisation der Fluoronderivate im Gewebe des Organismus nach einer bestimmten Zeit nach der Einführung des Präparats, da sich die Fluoronderivate innerhalb dieser Zeit zuerst in allen Geweben gleichmäßig verbreiten und erst danach in den bösartigen Geschwulsten akkumulieren. Das Austreten aus den normalen Geweben über die Leber und die Nieren beginnt praktisch sofort nach der Einführung des Präparats. Nach einer bestimmten Zeit, die für die Lokalisation, den Typ und die Größe der bösartigen Geschwulst charakteristisch ist, wird in der letzteren eine erhöhte Konzentration des Präparats festgestellt. Die Feststellung der Lokalisation der Fluoronderivate kann durch Fluoreszenzanalyse

und NMR-Tomografie erfolgen.

Die Gewebe mit einer erhöhten Konzentration des Fluorons wurden einer histologischen Analyse zwecks Belegung der bösartigen Natur des Gewebes unterworfen. Es wurde festgestellt, daß die Richtigkeit der Diagnostik eines bösartigen Vorgangs nach der Bestimmung der Fluoronderivate in den Geweben im Vergleich zu einer üblichen histologischen Analyse eines Biopsiematerials 75 bis 95 % in Abhängigkeit von der Lokalisation der Geschwulst beträgt.

Um das Wesen der Erfindung besser zu verstehen, sind in der Tabelle 1 Beispiele des verwendeten Mittels zur Kontrastierung von bösartigen Neubildungen angeführt.

Tabelle 1

Substituenten $R_1$ + $R_8$ in der allgemeinen Formel der Fluoronderivate

| Nr. der Verbindung | $R_1$ | $R_2$ | $R_3$ | $R_4$ | $R_5$ | $R_6$ | $R_7$ | $R_8$ |
|---|---|---|---|---|---|---|---|---|
| 1 | 2 | 3 | 4 | 5 | 6 | 7 | 8 | 9 |
| 1 | H | F | H | F | F | F | H | 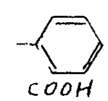 |
| 2 | H | H | $Na^+$ | H | H | F | H | 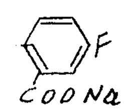 |
| 3 | OH | F | H | H | H | F | H | $CBr_3$ |
| 4 | $NH_2$ | H | H | H | H | H | $CF_3$ | 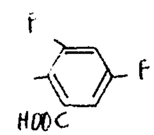 |
| 5 | H | H | $C_6H_5$ | H | H | COOH | $CH_3$ | 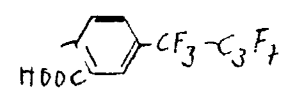 |

Tabelle 1 (Fortsetzung)

| | 1 | 2 | 3 | 4 | 5 | 6 | 7 | 8 |
|---|---|---|---|---|---|---|---|---|
| 6 | H | $NH_2$ | H | H | $CF_3$ | $COOC_2H_5$ | H | 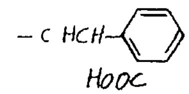 |
| 7 | $CF_3$ | H | $CH_3$ | H | H | H | $NH_2$ | 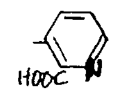 |
| 8 | H | H | H | $NO_2$ | H | H | H | 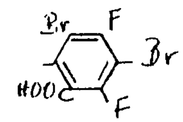 |
| 9 | H | $NO_2$ | H | COOH | H | $NO_2$ | COOH | 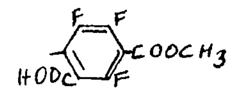 |
| 10 | $CBr_3$ | $CH_3$ | $CH_3$ | H | H | $C_6H_5$ | H | 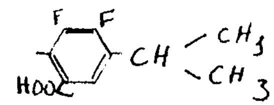 |
| 11 | H | H | H | H | H | $CBr_3$ | H | 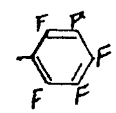 |
| 12 | H | $CH_3$ | $CH_3$ | $CH_3$ | $CH_3$ | $CH_3$ | H | 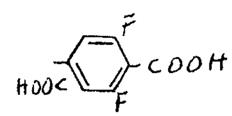 |

Tabelle 1 (Fortsetzung)

| 1 | 2 | 3 | 4 | 5 | 6 | 7 | 8 | 9 |
|---|---|---|---|---|---|---|---|---|
| 13 | $CH_3$ | OH | H | H | H | OH | $CH_3$ | 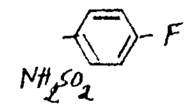 |
| 14 | H | $CF_3$ | H | H | H | $CF_3$ | H | 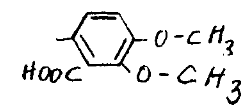 |
| 15 | H | H | H | $CF_3$ | $CF_3$ | H | H | 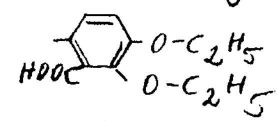 |
| 16 | H | F | H | $CF_3$ | $CF_3$ | F | H | 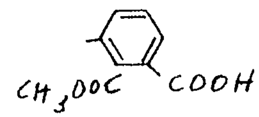 |
| 17 | F | F | H | $CH_3$ | $CH_3$ | F | F | 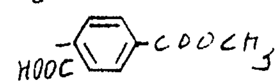 |
| 18 | $CH_3$ | H | H | J | J | H | H | 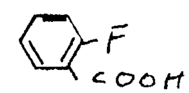 |
| 19 | H | OH | H | H | H | OH | H | 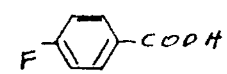 |

Tabelle 1 (Fortsetzung)

| 1 | 2 | 3 | 4 | 5 | 6 | 7 | 8 | 9 |
|---|---|---|---|---|---|---|---|---|
| 20 | $NO_2$ | COOH | H | H | H | COOH | H | 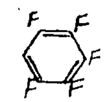 |
| 21 | H | COOH | H | $CF_3$ | $CF_3$ | COOH | H | 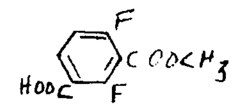 |
| 22 | H | H | H | $NH_2$ | $NH_2$ | H | H | 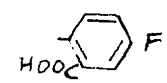 |
| 23 | H | H | H | 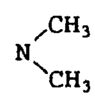 | 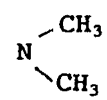 | H | H | 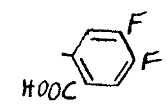 |
| 24 | H | $CH_3$ | H | $NH_2$ | $NH_2$ | $CH_3$ | H | 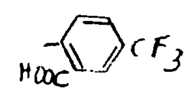 |
| 25 | F | F | H | F | H | COOH | H | 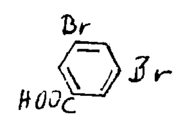 |
| 26 | H | $CH_3O$ | H | H | H | $CH_3O$ | H | 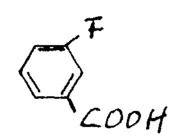 |

Tabelle 1 (Fortsetzung)

| 1 | 2 | 3 | 4 | 5 | 6 | 7 | 8 | 9 |
|---|---|---|---|---|---|---|---|---|
| 27 | H | $CH_3O$ | $CH_3$ | H | H | OH | H | 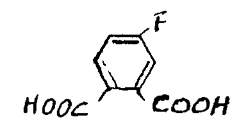 |
| 28 | H | $COOCH_3$ | H | H | H | $COOCH_3$ | H | 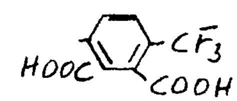 |
| 29 | H | COOH | H | $CH_3$ | $CH_3$ | COOH | H | 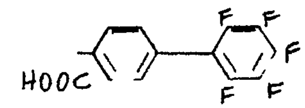 |

Tabelle 1 (Fortsetzung) Zusammensetzung des erfindungsgemäß verwendeten Mittels, in Gew.-%

| Nr. der Verbindung | Fluoronderivat | Glykose | Fruktose | Saccharose | Vitamine (s. Anlage zur Tabelle) Zusammensetzung I | Zusammensetzung II | Zusammensetzung III | Zusammensetzung IV | Antihistaminikum der Durchdringungsfähigkeit der Zellmembran 3-Methyl-9-benzyl-1,2,3,4-tetrahydrocarbonyl-naphthalin-1,5-disulfonat-18 | Kontrast der Akkumulierung des Fluoronderivats vats in der bösartigen Geschwulst im Vergleich zum normalen Gewebe in relativen Einheiten |
|---|---|---|---|---|---|---|---|---|---|---|
| 1 | 10 | 11 | 12 | 13 | 14 | 15 | 16 | 17 | 18 | 19 |
| 1 | 1,0 | 80,0 | - | - | - | - | 19,0 | - | - | 20,0 |
| 2 | 0,5 | - | 80,0 | - | - | - | - | 15,0 | 4,5 | 20,0 |
| 3 | 10,0 | 70,0 | - | - | 20,0 | - | - | - | - | 5,0 |
| 4 | 5,0 | - | 60,0 | - | 30,0 | - | - | - | 5,0 | 14,0 |
| 5 | 1,0 | - | - | 80,0 | - | - | - | 10,0 | 9,0 | 3,5 |

Tabelle 1 (Fortsetzung)

| 1 | 10 | 11 | 12 | 13 | 14 | 15 | 16 | 17 | 18 | 19 |
|---|---|---|---|---|---|---|---|---|---|---|
| 6 | 1,0 | - | - | - | - | - | - | 50,0 | 49,0 | 3,5 |
| 7 | 1,0 | - | 75,0 | - | - | 10,0 | - | - | 14,0 | 10,0 |
| 8 | 1,0 | 80,0 | - | - | 10,0 | - | - | - | 9,0 | 18,0 |
| 9 | 1,0 | 60,0 | - | - | 25,0 | - | - | - | 14,0 | 7,0 |
| 10 | 0,5 | - | - | 90,0 | - | 5,0 | - | - | 4,5 | 5,0 |
| 11 | 1,0 | 80,0 | - | - | - | - | 15,0 | - | 4,0 | 10,0 |
| 12 | 5,0 | - | - | 85,0 | - | - | - | - | 10,0 | 3,5 |
| 13 | 40,0 | 50,0 | - | - | - | - | - | - | 10,0 | 8,0 |
| 14 | 1,0 | 85,0 | - | - | - | - | - | 10,0 | 4,0 | 3,0 |
| 15 | 1,0 | 85,0 | - | - | - | - | - | 10,0 | 4,0 | 4,5 |

Tabelle 1 (Fortsetzung)

| 1 | 10 | 11 | 12 | 13 | 14 | 15 | 16 | 17 | 18 | 19 |
|---|---|---|---|---|---|---|---|---|---|---|
| 16 | 10,0 | - | 70,0 | - | - | - | - | 5,0 | 15,0 | 10,0 |
| 17 | 10,0 | - | 20,0 | - | 50,0 | - | - | - | 20,0 | 5,0 |
| 18 | 0,1 | 90,0 | - | - | - | - | 5,0 | - | 4,9 | 15,0 |
| 19 | 0,1 | 90,0 | - | - | - | - | 5,0 | - | 4,9 | 15,0 |
| 20 | 1,0 | - | 80,0 | - | - | 5,0 | - | - | 14,0 | 20,0 |
| 21 | 1,0 | - | - | 70,0 | - | - | - | 10,0 | 19,0 | 10,0 |
| 22 | 0,5 | - | 90,0 | - | - | - | - | 5,0 | 4,5 | 12,0 |
| 23 | 0,5 | - | - | 90,0 | - | - | 5,0 | - | 4,5 | 10,0 |
| 24 | 0,1 | 80,0 | - | - | 10,0 | - | - | - | 9,0 | 8,0 |
| 25 | 1,0 | 90,0 | - | - | - | 4,0 | - | - | 5,0 | 18,0 |

Tabelle 1 (Fortsetzung)

| 1 | 10 | 11 | 12 | 13 | 14 | 15 | 16 | 17 | 18 | 19 |
|---|---|---|---|---|---|---|---|---|---|---|
| 26 | 0,5 | 90,0 | - | - | - | - | - | - | 9,5 | 12,0 |
| 27 | 0,5 | - | 90,0 | - | 9,5 | - | - | - | - | 5,0 |
| 28 | 5,0 | - | 85,0 | - | - | - | 5,0 | - | 5,0 | 12,0 |
| 29 | 0,1 | 85,0 | - | - | - | 10.0 | - | - | 4,9 | 8,0 |

Anlage zur Tabelle 1

Zusammensetzung der Vitamine im erfindungsgemäß verwendeten Mittel, in Gew.-%

| | | |
|---|---|---|
| Zusammensetzung I: | A - Retinol | 1 % |
| | $B_1$ - Thiamin | 6 % |
| | $B_2$ - Riboflavin | 3 % |
| | $B_6$ - Pyridoxin-Chlorid | 6 % |
| | P - Rutin | 6 % |
| | E - Tocopherol-Acetat | 3 % |
| | C - Ascorbinsäure | 60 % |
| | PP - Nikotinsäure | 15 % |
| Zusammensetzung II: | $B_1$ | 5 % |
| | $B_2$ | 5 % |
| | P | 10 % |
| | E | 6 % |
| | C | 54 % |
| | PP | 20 % |
| Zusammensetzung III: | A | 2 % |
| | $B_2$ | 5 % |
| | P | 5 % |
| | E | 4 % |
| | C | 70 % |
| | PP | 14 % |
| Zusammensetzung IV: | $B_1$ | 8 % |
| | $B_2$ | 4 % |
| | P | 10 % |
| | C | 60 % |
| | PP | 18 % |

## Patentansprüche

1. Verwendung von Fluoronderivaten der allgemeinen Formel I

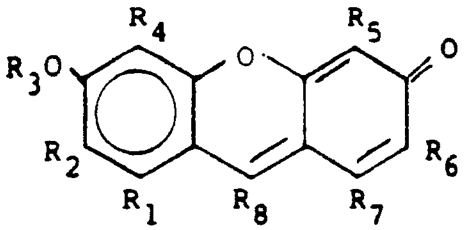

(I)

worin

$R_1$ und $R_7$ F oder einer F und der andere Wasserstoff (H), Cl, Br, J, $CH_3$, $CF_3$, $CCl_3$,$C_2H_5$, COOH, $COOCH_3$, $NO_2$, $NH_2$, OH, $OCH_3$ oder $OC_2H_5$ bedeuten;

$R_2$, $R_4$, $R_5$ und $R_6$ alle F oder eins bis drei F und der Rest ein oder mehrere aus der Gruppe Wasserstoff (H), Cl, Br, J, A, B, W, G, AW, AG, BW, BG, $CH_3$, $CF_3$, $CCl_3$, $CBr_3$, $CJ_3$, COOH, COOA, COOB, OA, OB, $NO_2$ und $NH_2$ bedeuten;

$R_3$ Wasserstoff (H), das Kation eines Alkalimetalls, Ammonium, A, B, W, G, Q, Z oder Glukoronsäure bedeutet;

$R_8$ = Wasserstoff (H) oder F oder F, welches in beliebiger Kombination in der Gruppe A, B, W, G, AW, AG, BW, BG, I, K, AI, BI, BK, AK, WI, WK, GI, GK, L, M, LM, Y , V, AY, BY, BV, AV, WY, GY, WV und GV für eine NMR-Kontrastierung von bösartigen Neubildungen vorliegt.

**2.** Verwendung von Fluoronderivaten der allgemeinen Formel I nach Anspruch 1 worin

$R_1$ und $R_7$ F oder einer F und der andere Wasserstoff (H), Cl, $CH_3$, $CF_3$, $NO_2$ oder $NH_2$ bedeuten;

$R_2$, $R_4$, $R_5$ und $R_6$ alle F oder einer bis drei F und der Rest ein oder mehrere aus der Gruppe Wasserstoff (H), $CF_3$, $NO_2$, $NH_2$ und OH bedeuten;

$R_3$ Wasserstoff (H), das Kation eines Alkalimetalls oder Ammonium bedeutet;

$R_8$ F oder die Gruppe A, substituiert durch F von eins bis zur vollständigen Substitution, die Gruppe B, substituiert durch F von eins bis zur vollständigen Substitution, die Gruppe I, substituiert durch F von eins bis zur vollständigen Substitution, die Gruppe Y, substituiert durch F, $CF_3$ von eins bis zur vollständigen Substitution, bei beliebiger Kombination unter ihnen, bedeutet, für eine NMR-Kontrastierung von bösartigen Neubildungen.

**3.** Anwendung von Fluoronderivaten der allgemeinen Formel I nach Anspruch 1, worin

$R_1$ und $R_7$ jeder mindestens eines aus der Gruppe Wasserstoff (H), F, Cl, $CH_3$, $CF_3$, $CCl_3$, COOH, $COOCH_3$, $NO_2$, $NH_2$, OH, $OCH_3$ oder $OC_2H_5$ bedeuten;

$R_2$, $R_4$, $R_5$ und $R_6$ aus der Gruppe Wasserstoff (H), F, Cl, die Gruppe A, sowie die Gruppe A, substituiert durch F, Cl von eins bis zur vollständigen Substitution bei beliebiger Kombination unter ihnen, die durch "P" bezeichnet ist, $CH_3$, $CF_3$, $CCl_3$, COOH, COOA, COOB, OA, OB, $NO_2$, $NH_2$ und W bedeuten;

Gruppe X die Gruppe W, substituiert von eins bis zur vollständigen Substitution durch F, Cl, $NO_2$, $NH_2$, OH, $HSO_3^-$, COOH oder $COO^-$ ausgewählt sind,

$R_3$ Wasserstoff (H), das Kation eines Alkalimetalls, Ammonium, A, P, W, X, Q oder Acyl (R-CH=O) bedeutet;

$R_8$ = Wasserstoff (H), A, P, W, X, AW, AX, PW, PX und I, Gruppe T die Gruppe I, substituiert durch F,

Cl, $NO_2$, $NH_2$, OH, $HSO_3^-$ , COOH, $COO^-$, von eins bis zur vollständigen Substituierung bei beliebiger Kombination unter ihnen, AI, PI, AT, PT, WI, XI, WT, XT, L, M, LM, Y, Gruppe U die Gruppe Y, substituiert durch $NO_2$, $NH_2$, OH, COOH, $COO^-$, $HS\overline{O}_3$, F, Cl, COOA, COOP,

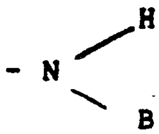 , 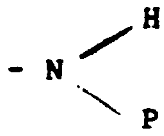 ,

$CH_3$, $CF_3$, $CCl_3$ von eins bis zur vollständigen Substitution in beliebiger Kombination unter ihnen, AY, PY, AU, PU, WY, XU, XY oder WU für eine Fluoreszenzkontrastierung von bösartigen Neubildungen sind.

**4.** Verwendung von Fluoronderivaten der allgemeinen Formel I nach Anspruch 3, worin

$R_1$ und $R_7$ aus der Gruppe Wasserstoff (H), F, Cl, $NO_2$, $NH_2$ und OH ausgewählt sind;

$R_2$, $R_4$, $R_5$ und $R_6$ aus der Gruppe Wasserstoff (H), F, Cl, $NO_2$, $NH_2$ und OH ausgewählt sind;

$R_3$ Wasserstoff (H), das Kation eines Alkalimetalls oder Ammoniums bedeutet;

$R_8$ Wasserstoff (H), die Gruppe W, X, I, T, Y, oder U für eine Fluoreszenzkontrastierung von bösartigen Neubildungen, bedeutet.